# EUROPÄISCHE PATENTSCHRIFT

(11) **EP 1 802 731 B1**
(45) Veröffentlichungstag und Bekanntmachung des Hinweises auf die Patenterteilung: **21.05.2008**
(21) Anmeldenummer: 05716082.2
(22) Anmeldetag: 15.03.2005
(51) Int. Cl.: C11C 3/10, C07C 67/03

(54) **VERFAHREN ZUR UMESTERUNG VON FETTEN UND ÖLEN BIOLOGISCHEN URSPRUNGS MITTELS ALKOHOLYSE UNTER VERWENDUNG SPEZIELLER KOHLENSÄURESALZE**
METHOD FOR THE TRANSESTERIFICATION OF FATS AND OILS OF BIOLOGICAL ORIGIN BY MEANS OF ALCOHOLYSIS USING SPECIFIC CARBONATES
PROCEDE DE TRANSESTERIFICATION DE GRAISSES ET D'HUILES D'ORIGINE BIOLOGIQUE AU MOYEN D'UNE ALCOOLYSE, A L'AIDE DE CARBONATES SPECIFIQUES

(30) Priorität: 15.09.2004 DE 102004044660
(43) Veröffentlichungstag der Anmeldung: 04.07.2007
(73) Patentinhaber: Peter, Dr., Wolfgang, 89075 Ulm (DE)
(72) Erfinder: PETER, Siegfried, 91080 Uttenreuth (DE); WEIDNER, Eckhard, 44795 Bochum (DE)
(74) Vertreter: Beyer, Andreas
(86) Internationale Anmeldenummer: PCT/EP2005/002752
(87) Internationale Veröffentlichungsnummer: WO 2006/029655

(56) Entgegenhaltungen:
- WO-A-01/29160
- WO-A-20/04031119
- SCHUCHARDT U ET AL: "TRANSESTERIFICATION OF VEGETABLE OILS: A REVIEW" JOURNAL OF THE BRAZILIAN CHEMICAL SOCIETY, SAO PAULO, BR, Bd. 9, Nr. 3, 1998, Seiten 199-210, XP009008364 ISSN: 0103-5053

## Beschreibung

Die Erfindung betrifft ein Verfahren zur Herstellung von Fettsäureestern aus Fetten und Ölen biologischen Ursprungs durch Umesterung mit einwertigen Alkoholen in Gegenwart von basischen Katalysatoren, wobei als Katalysatoren Salze aus einer basischen organischen Verbindung und Kohlensäure eingesetzt werden.

Eine kommerziell bedeutsame Klasse industrieller organischer Reaktionen sind Umesterungsreaktionen. Bei einer Umesterungsreaktion wird ein Ester durch Austausch der Säuregruppen oder durch Austausch der alkoholischen Gruppen in einen anderen Ester überführt. Erfolgt die Umesterung durch Austausch der alkoholischen Gruppen, so spricht man von einer Alkoholyse. Bei der Alkoholyse wird der auszutauschende Alkohol im allgemeinen im Überschuss zugesetzt, um eine hohe Ausbeute an gewünschtem Ester zu erhalten. In neuerer Zeit hat im Zusammenhang mit der Erzeugung von Dieselkraftstoff aus nachwachsenden Rohstoffen die Herstellung von Alkylestern, insbesondere von Methylestern aus vegetabilischen Ölen (z.B. Rapsöl, Sojaöl) erheblich an Aktualität gewonnen.

Die Umesterung ist eine Gleichgewichtsreaktion, die in der Regel bereits durch Mischen der Reaktanden ausgelöst wird. Die Reaktion verläuft jedoch so langsam, dass für kommerzielle Zwecke ein Katalysator zur Beschleunigung der Reaktion erforderlich ist. Fette und Öle biologischen Ursprungs bestehen überwiegend aus Glyceriden (Mono-, Di- und Triglycerid). In der Praxis wird häufig das Bradshaw-Verfahren zur Umesterung von Fetten und Ölen mit Methanol (beschrieben in den US-Patenten 2,271,619 und 2,360,844) angewandt. Die Reaktion wird in einem offenen Behälter, der aus gewöhnlichem Kohlenstoffstahl bestehen kann, durchgeführt. Hierbei wird das Fett oder Öl, dessen Säurezahl nicht über 1,5 liegen soll, bei der Siedetemperatur des Reaktionsgemisches mit einem Überschuss an 99,7 %-igem Methanol in Gegenwart von 0,1 bis 0,5 % Natriumhydroxid gerührt. Beim ruhigen Stehen scheidet sich das entstandene Glycerin praktisch wasserfrei am Boden des Gefäßes ab. Nach einer Stunde ist die Umsetzung meist 98 %. Das Fett oder Öl muss trocken (wasserfrei), sauber und vor allem neutral sein.

Werden bei der Umesterung von Triglyceriden mit Methanol und Ethanol Natrium- und Kaliumverbindungen als Katalysator verwendet, so treten verschiedene Probleme auf. Nach der teilweise erfolgten Umesterungsreaktion beginnt das erzeugte Glycerin, eine neue Phase zu bilden. Die Natrium- und Kaliumverbindungen als Katalysator sind in der Glycerinphase sehr gut löslich und verarmen entsprechend im Reaktionsgemisch. Deswegen und wegen der im Laufe der Reaktion gebildeten Emulsion schreitet die Reaktion schließlich nur langsam voran. Aus diesem Grunde wird häufig nach etwa einer halben Stunde die Reaktion unterbrochen und das gebildete Glycerin abgetrennt. Danach wird neuer Katalysator zugesetzt und die Reaktion eine weitere halbe Stunde fortgesetzt. Auf diese Weise wird ein Umsatz von etwa 98 % erhalten. In der Esterphase bleiben nach der Phasentrennung noch sehr feine Glycerintröpfchen suspendiert. Das Glycerin und der in beiden Phasen verteilte Katalysator müssen aus der Esterphase nach Abschluss der Reaktion entfernt werden. Je nach der weiteren Verwendung des Glycerins ist es darüber hinaus notwendig, den gelösten Katalysator auch aus der Glycerinphase zu entfernen. Um die genannten Probleme zu vermeiden und gleichzeitig die Reaktionszeit abzukürzen, wurden verschiedene Vorschläge gemacht.

In der deutschen Offenlegungsschrift DE 34 21 217 A1 wird ein Verfahren zur Herstellung von Fettsäureestern kurzkettiger, primärer und sekundärer Alkohole mit 1 bis 5 Kohlenstoffatomen durch Umesterung von Glyceriden beschrieben. Dabei wird durch die flüssigen Glyceride bei Temperaturen zwischen 230 und 270°C ein Strom des gasförmigen Alkohols hindurchgeleitet. Mit diesem Strom wird ein Produktionsgemisch aus Glycerin und Fettsäurealkylester aus der Reaktionszone ausgetragen und wird anschließend getrennt. Als Katalysator ist Alkali in den flüssigen Glyceriden des Reaktionsgefäßes gelöst.

Nach dem in der deutschen Patentschrift DE 198 03 053 C1 beschriebenen Verfahren werden Triglyceride in Gegenwart von geeigneten Katalysatoren, wie z. B. Zinkseifen, vorzugsweise in Gleichstromkolonnen bei Temperaturen von 200 bis 240°C und bei Drücken bis zu 90 bar mit Alkohol im mehrfach molaren Überschuss umgesetzt. Nach der Abtrennung des überschüssigen Alkohols werden Alkylester-Glycerin-Gemische erhalten, die in einem Separator in die leichtere organische Phase und in die Glycerinphase getrennt werden. An diese Phasentrennung schließt sich eine weitere Aufarbeitung und Reinigung der Produkte an. Die Esterphase wird mit Wasser gewaschen, um die im Produkt gelösten Glycerinreste zu entfernen. Dabei werden auch etwa 40 % der im Ester gelösten Zinkseifen in Form von Zinkhydroxid ausgewaschen.

Ferner wurden schon früher Untersuchungen mit dem Ziel angestellt, die Natrium- und Kaliumverbindungen durch basische Ammoniumverbindungen als Katalysator oder Reaktand zu ersetzen. Untersucht wurde im Hinblick auf ihre Eignung als Katalysatoren für die Alkoholyse von Fetten und Ölen die Aktivität zahlreicher Basen, z. B. Amine wie Triethylamin, 1,2,2,6,6-Pentamethylpyridin, 2,6-Di-tert-butylpyridin, 4-Dimethylaminopyridin (DMAP); 1,5,7-Triazabicyclo(4.4.0)dec-5-en (TBD), 1,1,3,3-Tetramethylguanidin (TMG), 1,2,3-Triphenylguanidin, 1,1,2,3,3-Pentabutylguanidin (PBG), 1,3-Diphenylguanidin, und weitere Amino- und Nitroguanidine; Triamino(imino)phosphorane wie tert-Butylamino-2-diethylamino-1,3-perhydro-1,2,3-diazaphoran (BEMP), Tris(dimethylamino)methyliminophosphoran (Me7P). Letztere werden häufig in der organischen Synthese verwendet. In einer Serie wurde die katalytische Aktivität einiger Guanidinverbindungen, z. B. der Amidine DBU und DBN und der Phosphorane BEMP und Me7P mit anderen Basen verglichen. Die Guanidine sind die aktiveren Katalysatoren. Die Aktivität folgt ihrer relativen Basizität. Die Aktivität von TBD bei einer Konzentration von 3 mol-% war ähnlich der von Kaliumcarbonat gleicher Konzentration.

Ein Vorteil, den Guanidine bei der Umesterung von Fetten und Ölen als Katalysator bieten, besteht in der Möglichkeit, sie auf organischen Polymeren zu binden. Schuchardt et al. untersuchten die Tauglichkeit von Cellulose, Polystyrol/Divinylbenzol, und Polyurethanen als Trägermaterial. Die Herstellung von heterogenen Katalysatoren durch Bindung von Guanidinen durch chemische Bindungskräfte an verschiedenen Polymeren und deren Eignung für die Katalyse der Umesterung von vegetabilischen Ölen und Fetten sind in dem brasilianischen Patent BR 8202429 (1984, Erfinder: U. Schuchardt und O.G. Lopes) beschrieben. Die Guanidine, die an gelartiges Poly(styrol/divinylbenzol) beziehungsweise Cellulose gebunden waren, zeigten gegenüber der katalytischen Reaktion in homogener Phase eine leicht verminderte Aktivität. Nach verlängerten Reaktionszeiten wurden aber gleich hohe Umsätze erreicht. Obgleich etwas weniger aktiv als die homogenen analogen Katalysatoren, konnten die heterogenen Katalysatoren in einer Reihe aufeinanderfolgenden Reaktionszyklen weiter Verwendung finden. Doch nahm die Aktivität schon nach 9 Reaktionszyklen merklich ab. Die Abnahme der Aktivität mit laufendem Einsatz wurde durch das langsame Auslaugen der verankerten Base aus dem Polymeren verursacht.

In der veröffentlichten internationalen Patentanmeldung WO 2004/031119 wird die Verwendung von basischen Katalysatoren ausgewählt aus Iminoverbindungen, Alkylguanidinen, Butylamin, quaternären Aminen und tertiären Aminen, die eine zusätzliche OH-Gruppe oder NH₂-Gruppe tragen, beschrieben.

In der veröffentlichten internationalen Patentanmeldung WO 01/12581 wird vorgeschlagen, in einem ersten Schritt eine Entsäuerung des pflanzlichen Öles oder Fettes durch Umsetzung der freien Fettsäuren mit Methanol und Schwefelsäure als Katalysator vorzunehmen. Nach der Neutralisation soll in einem zweiten Schritt die Umesterung unter Verwendung von Alkali (NaOH, KOH) stattfinden. Bei dem zweiten Schritt wird dem Reaktionsgemisch soviel an einem Cosolvent zugemischt, dass das Reaktionsgemisch einphasig wird. Dadurch wird die Reaktionsgeschwindigkeit erheblich erhöht. Als geeignetes Cosolvent werden Tetrahydrofuran, Methyl-tert-butylether, Pyridin und Bis(dimethylsilyl)trifluoroacetamid benannt.

Schuchardt et al offenbaren im Journal of the Brazilian Chemical Society, Bd. 9, Nr. 3, 1998, "Transesterification of Vegetable Oils: A Review" ein Verfahren zur Umesterung von pflanzlichen Öl mittels Alkoholyse, wobei ein einwertiges Alkohol und ein basischer Katalysator dem Öl zugegeben wird.

Aufgabe der vorliegenden Erfindung ist es, ein Verfahren zur Umesterung von Fett und/oder Öl bereitzustellen, das einfach auszuführen ist und die Alkylesterprodukte in hohen Ausbeuten ergibt.

Diese Aufgabe ist erfindungsgemäß mit einem Verfahren gelöst, das die im Patentanspruch 1 genannten Merkmale aufweist.

Überraschenderweise wurde demnach gefunden, dass Salze aus einer basischen organischen Verbindung und Kohlensäure aktive Katalysatoren für die Alkoholyse, insbesondere die Methanolyse, darstellen.

Verschiedene organische Basen, beispielsweise Guanidin, 1-Aminoguanidin, usw., bilden mit Kohlensäure gut kristallisierende Salze. Im kristallisierten Zustand sind die Salze gut zu handhaben und überdies stabil und lagerfähig sowie kostengünstig. Die Carbonate sind in Alkoholen und Fettsäureestern unlöslich, in Wasser wenig löslich. Die gesättigte wässrige Lösung von Guanidincarbonat hat einen pH-Wert von 11 bis 11,5. Dagegen reagiert die wässrige Lösung von freiem Guanidin ebenso stark alkalisch wie Alkali-Laugen. Versuchsweise wurde einem Gemisch aus 100 ml Rapsöl und 40 ml Methanol 2 g Guanidincarbonat zugegeben. Durch Rühren wurde eine stabile Suspension erhalten, die keine Besonderheit zeigte. Bei Erwärmen der Mischung bildeten sich bei Erreichen von Temperaturen im Bereich der Siedetemperatur des Methanols (60 bis 65°C) überraschenderweise Gasblasen, die aus Kohlendioxid bestanden. Es ist zwar bekannt, dass sich Alkalicarbonate bei hohen Temperaturen zersetzen. Je höher die Basizität des Kations, desto höher ist die Zersetzungstemperatur. In Anbetracht der hohen Basizität des Guanidins war der Zerfall des Guanidincarbonats bereits im Temperaturbereich des Siedepunktes des Methanols überraschend.

Die Zersetzung von Natriumhydrogencarbonat beginnt schon bei 100°C. Die Verbindung zerfällt in Natriumcarbonat, Kohlendioxid und Wasser. Eine für den praktischen Betrieb genügend schnelle Zersetzung erfordert jedoch Temperaturen von 170 bis 180°C. Die weitere Dissoziation des Natriumcarbonats setzt erst bei Überschreiten von Temperaturen von etwa 650°C ein. Im Vergleich dazu beginnt die Zersetzung des Carbonats des weniger basischen Calciums schon bei Temperaturen von 500°C. Mit wachsender Basizität steigt die Temperatur, bei der eine Dissoziation des jeweiligen Alkalicarbonats erfolgt. Ammoniumcarbonat zerfällt bei 58°C in Ammoniak und Kohlendioxid. Im Vergleich ist der Unterschied in der Basizität von Ammoniak und Guanidin sehr viel größer als der zwischen Calcium und Natrium. Umso überraschender ist die Beobachtung, dass die Dissoziation des Guanidincarbonats in Anwesenheit von Methanol bereits bei Temperaturen von ca. 60°C einsetzt und der große Unterschied in der Basizität zwischen Ammoniak und Guanidin in der Zersetzungstemperatur ihrer Carbonate keine Entsprechung erfährt.

Beim Sieden am Rücklauf, d.h. Verdampfen und anschließendes Kondensieren des Methanols, wird durch den Methanoldampf das Kohlendioxid aus dem Reaktionsgemisch abtransportiert und so das Dissoziationsgleichgewicht gestört, bis praktisch nur noch freies Guanidin vorhanden ist. Im Prinzip könnte auch vorgesehen werden, anstelle von Methanoldampf ein inertes Gas, wie beispielsweise Stickstoff, für den Abtransport des Kohlendioxids zu verwenden.

In einem Gemisch aus Ethanol und Rapsöl konnte selbst bei Temperaturen über 100°C noch keine Auflösung oder Gasbildung des Guanidincarbonats beobachtet werden. Offenbar ist die Dissoziation von Guanidincarbonat bei den niedrigen Temperaturen im Bereich der Siedetemperatur des Methanols auf eine spezielle Wechselwirkung zwischen Methanol und Guanidincarbonat zurückzuführen. In einem Gemisch aus Rapsöl mit einem Gemisch aus Methanol und Ethanol konnte erst eine merkliche Reaktion beobachtet werden, wenn der Methanolgehalt im Alkoholgemisch mindestens 30 Gew.-% betrug, vorzugsweise mehr als 50 Gew.-%, und insbesondere mehr als 70 Gew.-%.

Wird beispielsweise einem Reaktionsgemisch aus Rapsöl und Methanol etwa 1 Gew.-% eines pulverförmigen Kristallisats von Guanidincarbonat zugesetzt, so entsteht eine Suspension. Das Guanidincarbonat ist sowohl im Methanol als auch im Öl unlöslich. Wird die Suspension erwärmt, so entstehen bei Erreichen von etwa 60°C Gasbläschen, die aus Kohlendioxid bestehen. Mit dem Entweichen des Kohlendioxids entsteht freies, hoch basisches Guanidin, das die Bildung von Methylestern katalysiert. Mit Annäherung der Temperatur an die Siedetemperatur des Methanols wird die Gasbildung lebhafter. Das freie Guanidin ist in Methanol und in geringerem Maße auch in der Ölphase löslich. Lässt man das Reaktionsgemisch am Rücklauf sieden, so lässt nach etwa 10 Minuten die Gasbildung deutlich nach. Nach 60 Minuten ist in einem Prozessschritt ein Produkt durch die Reaktion von Öl mit Methanol entstanden, das der Europäischen Norm für Biodiesel, der DIN EN 14214 entspricht. Eine Unterbrechung der Reaktion, um entstandenes Glycerin auszuschleusen und mit neuem Katalysator die Reaktion zu Ende zu bringen, wie bei der Verwendung von Alkalien als Katalysator, ist nicht notwendig. Andere Öle, wie Sojaöl und Sonnenblumenöl führten zu analogen Ergebnissen. Zusammenfassend kann die beschriebene Reaktion als eine homogen katalysierte Phasentransfer-Reaktion bezeichnet werden.

Bei Sieden des Reaktionsgemisches aus Öl, Methanol und Guanidincarbonat bei Rücklauf entsteht aus dem Guanidincarbonat freies Guanidin, welches eine hohe Affinität zu H₂O hat und das bei der Bildung von Methylester aus Methanol und freien Fettsäuren entstehende H₂O binden kann. Die Wirkung bei dem Prozess ist dabei derjenigen der Schwefelsäure ähnlich, die gewöhnlich bei der Estersynthese aus Alkohol und Carbonsäuren verwendet wird. Da das entstehende Guanidinhydrat nur eine geringe katalytische Aktivität besitzt, muss dieser Anteil ersetzt werden. Praktisch kann das durch Erhöhung der Menge an Guanidincarbonat, die der Reaktionsmischung zu Beginn zugesetzt wird, geschehen.

Bei den vegetabilischen Ölen, wie Rapsöl, Sojaöl, Sonnenblumenöl usw. beträgt der Gehalt an freien Fettsäuren etwa 1 Gew.-%. Eine Menge von ca. 0,4 bis 0,5 Gew.-% Guanidincarbonat genügt, um das Wasser zu binden, das bei der Umsetzung dieser Menge an freien Fettsäuren entsteht. Das heißt, ein Zusatz von insgesamt 1,2 bis 1,3 g Guanidincarbonat würde genügen, um 100 g Öl innerhalb von 60 Minuten zu Biodiesel umzusetzen. Damit erübrigt sich eine Entfernung der freien Fettsäuren aus dem Edukt. Das entschleimte Öl kann somit in einem einzigen Verfahrensschritt in Methylester überführt werden, welcher die Europäische Norm für Biodiesel erfüllt.

Die vorliegende Erfindung ermöglicht folglich den Einsatz nicht-entsäuerter Öle mit beliebigen Anteilen freier Fettsäuren (z. B. 0,5; 1; 1,5; 2; 2,5; 3; 5, 10 Gew.-% und höher), die durch Zusatz entsprechend erhöhter Guanidincarbonatmengen (beispielsweise zusätzlich 0,2 bis 5 Gew.-%, vorzugsweise 0,4 bis 0,5 Gew.-%) effizient zu Methylester umgesetzt werden können.

Aus biotechnologischen Prozessen ist Ethanol leicht zugänglich und steht in großen Mengen zur Verfügung. Die unmittelbare Ethanolyse von Triacylglyceriden mit dem Zusatz von Guanidincarbonat als Katalysator ist jedoch bei tiefen Temperaturen nicht realisierbar. Überraschenderweise stellte sich nun heraus, dass Gemische aus Methanol und Ethanol, die einen Mindestanteil an Methanol enthalten, bei der Siedetemperatur des Reaktionsgemisches mit Hilfe von Guanidincarbonat als Katalysator umgesetzt werden können. Mit einem Alkoholgemisch aus gleichen Gewichtsteilen Ethanol und Methanol und Rapsöl konnte innerhalb von 60 Minuten beim Sieden am Rücklauf ein Produkt erzeugt werden, das der Europäischen Norm für Biodiesel entspricht. Freies Guanidin katalysiert die Ethanolyse ebenfalls.

Ein Reaktionsgemisch aus 100 ml Rapsöl, 40 ml Methanol und 2 g Guanidincarbonat wurde bei Rücklauf 60 Minuten auf Siedetemperatur gehalten und danach abgekühlt. Nach etwa 20 bis 30 Minuten waren im Reaktionsgemisch keine kristallinen Teilchen mehr zu beobachten. Nach Abkühlung hatten sich zwei Phasen abgetrennt. Die schwere Phase hatte ein Volumen von 20 ml; die leichte Phase ein Volumen von 118 ml. Nach Trennung der Phasen mit Hilfe eines Scheidetrichters wurde aus jeder Phase separat das Methanol durch Destillation entfernt. Danach betrug das Volumen der schweren Phase 10,5 ml, das der leichten Phase 104 ml. Die schwere Phase enthielt: 1,6 % Methylester, 0,1 % Monoglyceride, 98,2 % Glycerin. Die leichte Phase enthielt: 98,6 % Methylester, 0,5 % Monoglyceride, 0,1 % Diglyceride, 0,1 % Triglyceride, 0,4 % Sterine, 0,3 % Glycerin. Nach der europäischen Norm für Biodiesel sind 0,8 % Monoglyceride, 0,2 % Diglyceride und 0,2 % Triglyceride zugelassen.

Das als Katalysator wirkende freie Guanidin geht nach Ende der Reaktion in die schwere Phase über, in der die Hauptmenge des erzeugten Glycerins und überschüssiges Methanol gelöst sind.

Das Guanidinderivat 1-Aminoguanidin bildet ebenfalls mit Kohlensäure ein pulverförmiges Kristallisat. Im Bereich der Siedetemperatur des Methanols ist kaum ein Zerfall des 1 -Aminoguanidinhydrogencarbonats auszumachen. Erst bei höherer Temperatur läuft der Zerfall des Hydrogencarbonats mit größerer Geschwindigkeit ab. Das freie 1-Aminoguanidin ist ein Katalysator für die Methanolyse.

Bei den organischen basischen Verbindungen im Rahmen der vorliegenden Erfindung handelt es sich um stark basische organische Verbindungen, vorzugsweise um stickstoffhaltige Verbindungen. Bevorzugte Verbindungen sind Guanidin und 1-Aminoguanidin. Deren Kohlensäuresalze weisen den Vorteil auf, dass sie stabil und leicht handzuhaben sind, was die Durchführung des erfindungsgemäßen Verfahrens erleichtert.

Bevorzugt wird die Alkoholyse bei Temperaturen im Bereich von 50 bis 120°C, insbesondere 65 bis 85°C durchgeführt. Gemäß einer bevorzugten Ausführungsform findet die Reaktion unter Rückfluss und/oder kräftigem Rühren statt.

Bevorzugt wird die erfindungsgemäße Alkoholyse mit Methanol oder einer Mischung von Methanol und einem weiteren einwertigen Alkohol durchgeführt. Vorzugsweise beträgt die Methanolkonzentration in einem solchen Gemisch mehr als 30 Gew.-%, insbesondere mehr als 50 Gew.-%, und vorzugsweise mehr als 70 Gew.-%. Vorzugsweise wird Ethanol als weiterer einwertiger Alkohol in einem Gemisch eingesetzt.

Gemäß einer bevorzugten Ausführungsform kann das Guanidincarbonat in einem ersten Schritt separat in Methanol unter Abspaltung der Kohlensäure bei erhöhten Temperaturen gelöst werden und diese Lösung mit dem umzuesternden Fett und/oder Öl vermischt und zur Reaktion gebracht werden. Allgemein ist die katalytische Aktivität des freien Guanidins merklich höher als jene der Derivate des Guanidins.

Gemäß einer bevorzugten Ausführungsform wird ein Molverhältnis von Methanol zu Fett und/oder Öl von mindestens 2,3 zu 1, vorzugsweise von 3 zu 1 und insbesondere von 6 zu 1 oder höher angewendet.

Als Fette und/oder Öle eignen sich vegetabilische Öle wie beispielsweise Rapsöl, Palmöl oder Sojaöl sowie Fischöl, Schweinefett usw. Vorzugsweise enthalten die Fette/Öle einen Anteil an freien Fettsäuren im Ausgangsprodukt von nicht mehr als 0,2 %. Dies kann durch Entschleimen und Entsäuern der Ausgangsöle erreicht werden. Jedoch können die Fette und/oder Öle im Rahmen des vorliegenden Verfahrens auch ohne vorherige Entsäuerung eingesetzt werden, wie im Beispiel 7 unten dargestellt. Gemäß einer weiteren bevorzugten Ausführungsform wird unbehandeltes Öl, das etwa 1 Gew.-% bis 2 Gew.-% freie Fettsäuren enthält, mit zusätzlichem Guanidincarbonat zur Bindung des bei der Umsetzung der freien Fettsäuren entstehenden Wassers behandelt.

Zur weiteren Erläuterung der Erfindung sind im Folgenden einige Beispiele angeführt.

### Beispiel 1

Ein Gemisch aus 100 ml (92 g) Rapsöl, 42 ml (33 g) Methanol und 0,7 g Guanidincarbonat wurde unter Rühren bei Rücklauf 60 Minuten zum Sieden erhitzt. Nach Unterbrechung der Reaktion und Abkühlen des Reaktionsgemisches entstanden zwei flüssige Phasen. Die obere, leichte Phase hatte ein Volumen von 116 ml. Die schwere Phase hatte ein Volumen von 24 ml. Durch Abdestillieren des Methanols unter Wasserstrahlvakuum wurde das Volumen der schweren Phase auf 7 ml Flüssigkeit mit der Zusammensetzung: 98,2 % Glycerin, 1,6 % Methylester, und 0,1 % Monoglyceride reduziert. Das entstandene Glycerin enthält die Hauptmenge des zugegebenen Guanidins und ist deswegen stark alkalisch. Die leichte Phase hatte nach Abdestillieren des Methanols bei Wasserstrahlvakuum ein Volumen von 106 ml. Das Produkt enthielt abzüglich Glycerin (0,3 %): 98,6 % Methylester, 0,7 % Monoglyceride, 0,2 % Diglyceride, 0,5 % Sterine (Unverseifbares).

### Beispiel 2

Ein Gemisch aus 100 ml (92 g) Rapsöl, 40 ml (31 g) Methanol, und 1 g Guanidincarbonat wurde unter Rühren bei Rücklauf 30 Minuten zum Sieden erhitzt. Nach Unterbrechung der Reaktion und Abkühlen des Reaktionsgemisches entstanden zwei flüssige Phasen. Die leichte Phase hatte ein Volumen von 118 ml. Die schwere Phase hatte ein Volumen von 22 ml. Durch Abdestillieren des Methanols bei Wasserstrahlvakuum wurde das Volumen der schweren Phase auf 8,5 ml Flüssigkeit mit der Zusammensetzung: 97,3% Glycerin, 2,4 % Methylester und 0,1 % Monoglyceride reduziert. Das entstandene Glycerin ist stark alkalisch. Die leichte Phase hatte nach Abdestillieren des Methanols bei Wasserstrahlvakuum ein Volumen von 105 ml. Das Produkt enthielt: 98,5 % Methylester, 0,5 % Monoglyceride, 0,1 % Diglyceride, 0,1 % Triglyceride, 0,4 % Sterine (Unverseifbares), 0,4 % Glycerin.

### Beispiel 3

Ein Gemisch aus 100 ml Rapsöl, 40 ml Methanol, und 0,5 g Guanidincarbonat wurde unter Rühren bei Rücklauf 60 Minuten zum Sieden erhitzt. Nach Unterbrechung der Reaktion und Abkühlen des Reaktionsgemisches entstanden zwei flüssige Phasen. Die leichte Phase hatte ein Volumen von 116 ml. Die schwere Phase hatte ein Volumen von 21,5 ml. Durch Abdestillieren des Methanols bei Wasserstrahlvakuum wurde das Volumen der schweren Phase auf 8 ml mit der Zusammensetzung: 9 8,7 % Glycerin, 1,2 % Methylester, 0,1 % Monoglyceride reduziert. Das entstandene Glycerin ist stark alkalisch. Die leichte Phase hatte nach Abdestillieren des Methanols bei Wasserstrahlvakuum ein Volumen von 107 ml. Das Produkt enthielt: 98,3 % Methylester, 0,6 % Monoglyceride, 0,2 % Diglyceride, 0,1 % Triglyceride, 0,4 % Sterine (Unverseifbares), 0,4 % Glycerin.

### Beispiel 4

Ein Gemisch aus 100 ml Rapsöl, 40 ml Methanol, und 1 g 1-Aminoguanidinhydrogencarbonat wurde 60 Minuten unter Rühren bei Rücklauf zum starken Sieden erhitzt (die Siedetemperatur war dadurch etwa 0,5 bis 1°C höher als in den Beispielen 1 bis 3). Nach Unterbrechung der Reaktion und Abkühlen des Reaktionsgemisches entstanden zwei flüssige Phasen. Die leichte Phase hatte ein Volumen von 64 ml. Die schwere Phase hatte ein Volumen von 76 ml. Die leichte Phase hatte nach Abdestillieren des Methanols bei Wasserstrahlvakuum ein Volumen von 50 ml. Das Produkt enthielt: 36,7 % Methylester, 4,8 % Monoglyceride, 27,8 % Diglyceride, 29,5 % Triglyceride, 0,7 % Sterine, 0,5 % Glycerin.

### Beispiel 5

100 ml Rapsöl, 20 ml Ethanol, 20 ml Methanol und 1 g pulverförmiges, kristallines Guanidincarbonat wurden in einen Behälter gegeben und unter Rühren 60 Minuten am Rücklauf zum Sieden erhitzt. Anschließend wurde das Reaktionsgemisch auf Umgebungstemperatur abgekühlt. Es entstanden zwei Phasen. Die schwere Phase hatte ein Volumen von ca. 16 ml; die leichte Phase hatte ein Volumen von ca. 123 ml. Abzüglich Lösungsmittel enthielt die schwere Phase: 7,6 % Ester, 92,4 % Glycerin. Die leichte Phase enthielt abzüglich überschüssige Alkohole: 98,7 % Ester, 0,3 % Monoglyceride, 0,2 % Diglyceride, 0,1 % Triglyceride, 0,4 % Sterole, 0,3 % Glycerin.

### Beispiel 6

100 ml Sojaöl, 40 ml Methanol und 1 g Guanidincarbonat wurden 60 Minuten unter Rühren bei Rücklauf zum Sieden erhitzt. Nach Unterbrechung der Reaktion und Abkühlen des Reaktionsgemisches entstanden zwei flüssige Phasen. Die leichte Phase hatte ein Volumen von 116 ml. Die schwere Phase hatte ein Volumen von 21 ml. Nach Abdestillieren des überschüssigen Methanols bei Wasserstrahlvakuum hatte die leichte Phase ein Volumen von 106 ml. Das Produkt enthielt: 99,0 % Methylester, 0,7 % Monoglyceride, 0,3 % Sterole und Tocopherole. Das Volumen der schweren Phase wurde durch Abdestillieren bei Wasserstrahlvakuum auf 7,5 ml reduziert. Die Zusammensetzung der eingedampften schweren Phase betrug: 98,0 % Glycerin, 1,9 % Methylester, 0,1 % Monoglyceride.

### Beispiel 7

100 ml Palmöl (mit einem Gehalt an etwa 1,5 % freien Fettsäuren), 40 ml Methanol und 1 g pulverförmiges, kristallines Guanidincarbonat wurden in einen Behälter gegeben und unter Rühren 60 Minuten bei Rücklauf zum Sieden erhitzt. Nach Abbrechen der Reaktion und Abkühlen des Reaktionsgemisches auf 30°C entstanden zwei flüssige Phasen. Die schwere Phase hatte ein Volumen von 23,5 ml. Die leichte Phase hatte ein Volumen von 115 ml. Die schwere Phase enthielt abzüglich Lösemittel: 96,6 % Glycerin, 1,6 % Methylester, 0,6 % Monoglyceride, 0,2 % Diglyceride, 0,9 % freie Fettsäuren. Die leichte Phase enthielt abzüglich Lösemittel: 94,8 % Methylester, 1,3 % Monoglyceride, 1,5 % Diglyceride, 0,7 % Triglyceride, 1,1 % freie Fettsäuren, 0,6 % Glycerin.

## Patentansprüche

1. Verfahren zur Umesterung von Fett und/oder Öl biologischen Ursprungs mittels Alkoholyse, mit den Schritten:
- umzuesternden Fetts und/oder Öls biologischen Ursprungs in einem Gefäß Bereitstellen, und
- Zugeben eines einwertigen Alkohols und eines Katalysators zu dem bereitgestellten Fett und/oder Öl, **dadurch gekennzeichnet, dass** der Katalysator ein Salz aus einer basischen organischen Verbindung und Kohlensäure ist.

2. Verfahren nach Anspruch 1, **dadurch gekennzeichnet, dass** die basische Verbindung eine stickstoffhaltige Verbindung ist.

3. Verfahren nach Anspruch 1 oder 2, **dadurch gekennzeichnet, dass** die basische Verbindung Guanidin ist.

4. Verfahren nach Anspruch 3, **dadurch gekennzeichnet, dass** der Katalysator Guanidincarbonat ist.

5. Verfahren nach Anspruch 1 oder 2, **dadurch gekennzeichnet, dass** die basische Verbindung ein Derivat von Guanidin ist.

6. Verfahren nach Anspruch 5, **dadurch gekennzeichnet, dass** das Derivat von Guanidin 1-Aminoguanidin ist.

7. Verfahren nach Anspruch 6, **dadurch gekennzeichnet, dass** der Katalysator 1-Aminoguanidinhydrogencarbonat ist.

8. Verfahren nach einem der Ansprüche 1 bis 7, **dadurch gekennzeichnet, dass** das Carbonat oder Hydrogencarbonat der basischen Verbindung bei Erreichen der Siedetemperatur des Reaktionsgemisches in die Base und Kohlensäure zerfällt.

9. Verfahren nach Anspruch 8, **dadurch gekennzeichnet, dass** als einwertiger Alkohol Methanol verwendet wird.

10. Verfahren nach einem der Ansprüche 1 bis 8, **dadurch gekennzeichnet, dass** als einwertiger Alkohol ein Gemisch aus Methanol und einem weiteren einwertigen Alkohol verwendet wird.

11. Verfahren nach Anspruch 10, **dadurch gekennzeichnet, dass** als einwertiger Alkohol ein Gemisch aus Methanol und Ethanol verwendet wird.

12. Verfahren nach Anspruch 11, **dadurch gekennzeichnet, dass** ein Gemisch aus Methanol und Ethanol verwendet wird, bei dem die Methanolkonzentration größer als 30 Gew.-% ist.

13. Verfahren nach einem der Ansprüche 1 bis 12, **dadurch gekennzeichnet, dass** die Alkoholyse bei Temperaturen im Bereich von 50 bis 120°C, insbesondere im Bereich von 65 bis 80° C, durchgeführt wird.

14. Verfahren nach einem der Ansprüche 1 bis 13, **dadurch gekennzeichnet, dass** das Reaktionsgemisch während der Reaktion bei Rücklauf siedet.

15. Verfahren nach einem der Ansprüche 1 bis 14, **dadurch gekennzeichnet, dass** das im Reaktionsgemisch freigesetzte Kohlendioxid durch Hindurchleiten eines inerten Gases abgeführt wird.

16. Verfahren nach einem der Ansprüche 1 bis 15, **dadurch gekennzeichnet, dass** der Anteil freier Fettsäuren in dem bereitgestellten Fett bis zu 2 Gew.-% beträgt.

17. Verfahren nach einem der Ansprüche 1 bis 15, **dadurch gekennzeichnet, dass** der Anteil freier Fettsäuren in dem bereitgestellten Fett 0,2 % nicht überschreitet.

18. Verfahren nach einem der Ansprüche 1 bis 16, **dadurch gekennzeichnet, dass** das Reaktionsgemisch während der Reaktion kräftig gerührt wird.

19. Verfahren nach einem der Ansprüche 1 bis 17, **dadurch gekennzeichnet, dass** das Guanidincarbonat in einem ersten Schritt separat in Methanol unter Abspaltung der Kohlensäure bei erhöhten Temperaturen gelöst wird und die Lösung dann mit dem umzuesternden Fett und/oder Öl vermischt und zur Reaktion gebracht wird.

20. Verfahren nach einem der Ansprüche 1 bis 18, **dadurch gekennzeichnet, dass** das Molverhältnis von Methanol zu Fett und/oder Öl mindestens 2,3 zu 1 beträgt.

## Claims

1. Process for the transesterification of fat and/or oil of biological origin by means of alcoholysis, comprising the steps of:
- providing the fat and/or oil of biological origin to be transesterified in a vessel, and
- adding a monohydric alcohol and a catalyst to the provided fat and/or oil, **characterized in that** the catalyst is a salt of a basic organic compound and carbonic acid.

2. Process according to Claim 1, **characterized in that** the basic compound is a nitrogen-containing compound.

3. Process according to Claim 1 or 2, **characterized in that** the basic compound is guanidine.

4. Process according to Claim 3, **characterized in that** the catalyst is guanidine carbonate.

5. Process according to Claim 1 or 2, **characterized in that** the basic compound is a guanidine derivative.

6. Process according to Claim 5, **characterized in that** the guanidine derivative is 1-aminoguanidine.

7. Process according to Claim 6, **characterized in that** the catalyst is 1-aminoguanidine hydrogencarbonate.

8. Process according to one of Claims 1 to 7, **characterized in that** the carbonate or hydrogencarbonate of the basic compound decomposes into the base and carbonic acid on reaching the boiling point of the reaction mixture.

9. Process according to Claim 8, **characterized in that** methanol is used as the monohydric alcohol.

10. Process according to one of Claims 1 to 8, **characterized in that** a mixture of methanol and another monohydric alcohol is used as the monohydric alcohol.

11. Process according to Claim 10, **characterized in that** a mixture of methanol and ethanol is used as the monohydric alcohol.

12. Process according to Claim 11, **characterized in that** a mixture of methanol and ethanol is used in which the methanol concentration is greater than 30 wt.%.

13. Process according to one of Claims 1 to 12, **characterized in that** the alcoholysis is carried out at temperatures ranging from 50 to 120°C and especially from 65 to 80°C.

14. Process according to one of Claims 1 to 13, **characterized in that** the reaction mixture boils during the reaction while refluxing.

15. Process according to one of Claims 1 to 14, **characterized in that** the carbon dioxide liberated in the reaction mixture is entrained by the passage of an inert gas.

16. Process according to one of Claims 1 to 15, **characterized in that** the proportion of free fatty acids in the starting fat is up to 2 wt.%.

17. Process according to one of Claims 1 to 15, **characterized in that** the proportion of free fatty acids in the starting fat does not exceed 0.2%.

18. Process according to one of Claims 1 to 16, **characterized in that** the reaction mixture is stirred vigorously during the reaction.

19. Process according to one of Claims 1 to 17, **characterized in that** in a first step the guanidine carbonate is separately dissolved in methanol at elevated temperatures, with the elimination of carbonic acid, and the solution is then mixed and reacted with the fat and/or oil to be transesterified.

20. Process according to one of Claims 1 to 18, **characterized in that** the molar ratio of methanol to fat and/or oil is at least 2.3 to 1.

## Revendications

1. Procédé de transestérification de graisses et/ou d'huiles d'origine biologique au moyen d'une alcoolyse, comprenant pour étapes :
- la mise à disposition dans un récipient d'une graisse et/ou d'une huile d'origine biologique à transestérifier
- l'addition d'un alcool monovalent et d'un catalyseur à la graisse et/ou à l'huile mis à disposition, **caractérisé en que** le catalyseur est un sel formé d'un composé organique basique et d'acide carbonique.

2. Procédé selon la revendication 1, **caractérisé en ce que** le composé basique est un composé azoté.

3. Procédé selon la revendication 1 ou 2, **caractérisé en ce que** le composé basique est de la guanidine.

4. Procédé selon la revendication 3, **caractérisé en ce que** le catalyseur est un carbonate de guanidine.

5. Procédé selon la revendication 1 ou 2, **caractérisé en ce que** le composé basique est un dérivé de guanidine.

6. Procédé selon la revendication 5, **caractérisé en ce que** le dérivé de guanidine est de la 1-aminoguanidine.

7. Procédé selon la revendication 6, **caractérisé en ce que** le catalyseur est un hydrogénocarbonate de 1-aminoguanidine.

8. Procédé selon l'une des revendications 1 à 7, **caractérisé en ce que** le carbonate ou l'hydrogénocarbonate du composé basique se décompose en base et en acide carbonique lorsqu'est atteinte la température d'ébullition du mélange de réaction.

9. Procédé selon la revendication 8, **caractérisé en ce que** du méthanol est utilisé comme alcool monovalent.

10. Procédé selon l'une des revendications 1 à 8, **caractérisé en ce qu'**un mélange formé de méthanol et d'un autre alcool monovalent est utilisé comme alcool monovalent.

11. Procédé selon la revendication 10, **caractérisé en ce qu'**un mélange formé de méthanol et d'éthanol est utilisé comme alcool monovalent.

12. Procédé selon la revendication 11, **caractérisé en ce qu'**il est utilisé un mélange formé de méthanol et d'éthanol, dans le cadre duquel la concentration de méthanol est supérieure à 30 % en poids.

13. Procédé selon l'une des revendications 1 à 12, **caractérisé en ce que** l'alcoolyse est effectuée à des températures situées dans une plage comprise entre 50 et 120°C, plus particulièrement dans une plage comprise entre 65 et 80°C.

14. Procédé selon l'une des revendications 1 à 13, **caractérisé en ce que** le mélange de réaction bout au reflux pendant la réaction.

15. Procédé selon l'une des revendications 1 à 14, **caractérisé en ce que** le dioxyde de carbone libéré dans le mélange de réaction est évacué par passage d'un gaz inerte.

16. Procédé selon l'une des revendications 1 à 15, **caractérisé en ce que** la concentration d'acides gras libres dans la graisse mise à disposition peut atteindre jusqu'à 2% en poids.

17. Procédé selon l'une des revendications 1 à 15, **caractérisé en ce que** la concentration d'acides gras libres dans la graisse mise à disposition ne dépasse pas 0,2%.

18. Procédé selon l'une des revendications 1 à 16, **caractérisé en ce que** le mélange de réaction est fortement agité pendant la réaction.

19. Procédé selon l'une des revendications 1 à 17, **caractérisé en ce que** le carbonate de guanidine est dans un premier temps dissous séparément dans du méthanol à des températures élevées, avec séparation de l'acide carbonique, et que la solution est ensuite mélangée à la graisse et/ou à l'huile à transestérifier puis mise en réaction.

20. Procédé selon l'une des revendications 1 à 18, **caractérisé en ce que** le rapport molaire méthanol:graisse et/ou méthanol:huile est au moins de 2,3 : 1.
